# FASCICULE DE BREVET EUROPEEN

(11) **EP 1 351 655 B1**
(45) Date de publication et mention de la délivrance du brevet: **13.12.2006**
(21) Numéro de dépôt: 02710979.2
(22) Date de dépôt: 11.01.2002
(51) Int. Cl.: A61K 8/81, A61Q 5/12

(54) **COMPOSITION CONTENANT AU MOINS UN COMPOSE INSOLUBLE DANS L'EAU PARTICULIER ET AU MOINS UN POLYMERE AMPHIPHILE**
ZUSAMMENSTZUNG ENTHALTEND MINDESTENS EINE BESTIMMTE WASSERUNLÖSLICHE VERBINDUNG UND MINDESTENS EIN AMPHIPHILES POLYMER
COMPOSITION CONTAINING AT LEAST ONE WATER-INSOLUBLE COMPOUND AND AT LEAST ONE AMPHIPHILIC POLYMER

(30) Priorité: 11.01.2001 FR 0100333
(43) Date de publication de la demande: 15.10.2003
(73) Titulaire: L'ORÉAL, 75008 Paris (FR)
(72) Inventeur: FACK, Géraldine, F-92300 Levallois-Perret (FR); RESTLE, Serge, F-95390 Saint-Prix (FR)
(74) Mandataire: Le Blainvaux Bellegarde, Françoise
(86) Numéro de dépôt international: PCT/FR2002/000103
(87) Numéro de publication internationale: WO 2002/055033

(56) Documents cités:
- EP-A- 0 406 042
- EP-A- 0 750 899
- EP-A- 0 815 843
- EP-A- 1 055 406
- WO-A-00/31154
- US-A- 4 318 901
- US-A- 4 460 732
- US-A- 4 861 499
- US-A- 5 089 578
- US-A- 5 114 706
- US-A- 5 464 452
- A KOBAYASHI ET ALL: "Solubilization Properties of N-substituted Amphiphilic Acrylamide Copolymers" JOURNAL OF APPLIED POLYMER SCIENCE., vol. 73, no. 12, 19 septembre 1999 (1999-09-19), pages 2447-2453, XP002177425 JOHN WILEY AND SONS INC. NEW YORK., US ISSN: 0021-8995
- PATENT ABSTRACTS OF JAPAN vol. 1997, no. 02, 28 février 1997 (1997-02-28) & JP 08 252447 A (SHISEIDO CO LTD), 1 octobre 1996 (1996-10-01)

## Description

La présente invention se rapporte à une composition pour application topique, comprenant au moins un composé insoluble dans l'eau particulier et au moins un polymère amphiphile, et à son utilisation notamment pour le traitement et le soin de la peau humaine, du cuir chevelu, des muqueuses, des ongles et des cheveux.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légéreté du cheveu), un manque de lissage (cheveu non homogène de la racine à la pointe).

De plus, compte tenu du caractère généralement insoluble des agents conditionneurs utilisables dans les compositions cosmétiques en particulier pour les cheveux, on cherche à maintenir les agents conditionneurs en dispersion régulière dans le milieu sans cependant faire chuter la viscosité et les propriétés des compositions.
Les composés insolubles tels que les agents conditionneurs doivent également être véhiculées sur les matières kératiniques traitées en vue de leur conférer, suivant l'application, des propriétés de douceur, de brillance et de démêlage.

Il existe à ce jour peu de moyens pour maintenir efficacement en suspension les composés insolubles, car il s'agit là d'un problème difficile à résoudre ; à cet effet, on a déjà proposé l'utilisation des dérivés d'esters ou d'éthers à longue chaîne (agents nacrants) ou des polysaccharides tels que la gomme de xanthane (gélifiants). Cependant, les agents nacrants présentent des problèmes de cristallisation qui entraînent une évolution (augmentation) de la viscosité des compositions au cours du temps ; les agents gélifiant présentent également des inconvénients, à savoir, d'une part que la mousse des compositions détergentes contenant de la gomme de xanthane se développe difficilement (mauvais démarrage de mousse) et que, d'autre part, les compositions n'ont pas une texture lisse et s'écoulent par paquets, ce qui est peu apprécié des utilisateurs.

En outre, ces compositions laissent la peau « comme mouillée » après application, ces compositions ne pénétrant pas suffisamment dans la peau. Tout ceci rend leur utilisation rédhibitoire dans les domaines cosmétique et/ou dermatologique.

Par ailleurs, les copolymères anioniques réticulés comportant des groupements 2-acrylamido 2-méthyl propane sulfonique, tels que le produit commercialisé par la société SEPPIC sous la dénomination SEPIGEL 305 (constitué de motifs dérivant de la réaction entre (i) l'acrylamide, (ii) l'acide 2-acrylamido 2-méthyl propane sulfonique et (iii) au moins un composé à polyinsaturation oléfinique) ne permettent pas d'obtenir des compositions se répartissant de façon homogène et présentant une bonne rinçabilité et un bon dépôt de l'agent conditionneur sur les matières kératiniques .

En outre, si l'utilisation des homopolymères d'acide 2-acrylamido 2-méthylpropane sulfonique (AMPS), tels que le produit commercialisé par la société Clariant sous la dénomination Hostacerin AMPS permet d'obtenir la gélification de milieux aqueux tout en gardant de bonnes propriétés cosmétiques, par rapport à d'autres gélifiants disponibles, les compositions se répartissent mal sur l'ensemble de la chevelure et se rincent également difficilement.

II subsiste donc le besoin de compositions contenant des agents conditionneurs insolubles et ne présentant pas les inconvénients de l'art antérieur.

La demanderesse a trouvé de façon inattendue un polymère amphiphile permettant d'une part de stabiliser les compositions contenant des composés insolubles particuliers, notamment sans entraîner la fluidification ou l'épaississement des dites compositions ou le relarguage dudit composé insoluble et d'autre part d'obtenir des compositions se répartissant facilement sur les cheveux, se rinçant facilement sans laisser un toucher désagréable sur les cheveux. Par ailleurs, l'agent insoluble se dépose particulièrement bien sur les matières kératiniques notamment les cheveux.

Aussi, la présente invention a pour objet une composition pour application topique, comportant une phase aqueuse comprenant au moins un polymère amphiphile comportant au moins un monomère à insaturation éthylénique à groupement sulfonique, sous forme libre ou partiellement ou totalement neutralisée et comprenant au moins une partie hydrophobe au moins un composé insoluble dans l'eau choisi parmi :
I) les huiles de synthèse
II) les cires végétales et les cires synthétiques choisies parmi les cires de polyéthylène, de polyoléfines ou de Fischer-Tropsch,
III) les huiles végétales choisies parmi les huiles de tournesol, de maïs, de soja, d'avocat, de courge, de pépins de raisin, de sésame, de noisette, l'huile de Purcellin, les huiles essentielles naturelles ou synthétiques
IV) les esters d'acides di, tri ou tétracarboxyliques ,les monoesters d'acides monocarboxyliques aliphatiques saturés ou insaturés, linéaires ou ramifiés en C₁-C₅ et d'alcools ou de polyols aliphatiques saturés ou insaturés, linéaires ou ramifiés en C₁-C₂₆, les monoesters d'acides monocarboxyliques aliphatiques saturés ou insaturés, linéaires ou ramifiés en C₁-C₂₆ et d'alcools aliphatiques saturés ou insaturés, linéaires ou ramifiés en C₁-C₅,
V) les pigments non filtrants, les nacres et les charges
VI) les composés de type céramide répondant à la formule générale (IV): 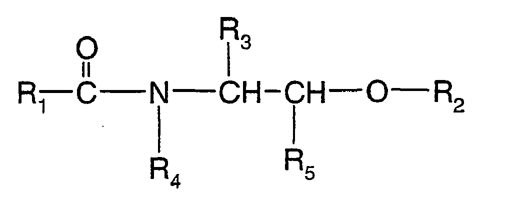
dans laquelle :
- R₁ désigne :
   - soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C₃₅, le ou les hydroxyles du radical R₇ pouvant être estérifié par un acide gras saturé ou insaturé, linéaire ou ramifié, éventuellement mono ou polyhydroxylé, en C₁-C₃₅;
   - soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent.
   - soit un radical R₈-O-CO-(CH2)ₚ, R₈ désigne un radical hydrocarboné en C₁-C₂₀, p est un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄ ; de préférence, R₃ désigne un radical α-hydroxyalkyle en C₁₅-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH₂)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle,

Les compositions selon l'invention ne contiennent de préférence pas de colorants directs des fibres kératiniques, de colorants d'oxydation des fibres kératiniques, d'agents réducteurs et d'agents oxydants.

La composition de l'invention étant destinée à une application topique, elle comprend un milieu physiologiquement acceptable. On entend dans la présente demande par « milieu physiologiquement acceptable » un milieu compatible avec toutes les matières kératiniques telles que la peau y compris le cuir chevelu, les ongles, les muqueuses, les yeux et les cheveux ou toute autre zone cutanée du corps.

La composition obtenue présente une excellente stabilité et conserve une viscosité satisfaisante (sans fluidication ou épaississement incontrôlé ou relargage du composé insoluble), même en présence d'une quantité importante de ce composé.

Aussi, la présente invention a également pour objet l'utilisation d'un polymère amphiphile comportant au moins un monomère à insaturation éthylènique à groupement sulfonique, sous forme libre ou partiellement ou totalement neutralisée et comprenant au moins une partie hydrophobe, pour stabiliser une composition cosmétique ou dermatologique contenant au moins un composé insoluble dans l'eau tel que défini ci-dessus et/ou améliorer son dépôt.

Un autre objet de l'invention concerne l'utilisation d'un polymère amphiphile comportant au moins un monomère à insaturation éthylènique à groupement sulfonique, sous forme libre ou partiellement ou totalement neutralisée et comprenant au moins une partie hydrophobe, dans, ou pour la fabrication d'une composition cosmétique comprenant au moins un composé insoluble dans l'eau tel que défini ci-dessus.

Les différents objets de l'invention vont maintenant être détaillés. L'ensemble des significations et définitions des composés utilisés dans la présente invention données ci-dessous sont valables pour l'ensemble des objets de l'invention.

La composition obtenue a la viscosité souhaitée, c'est-à-dire généralement supérieure à 0,5 poise, par exemple de 1 à 50 poises (0,1 à 5 Pa.s), et de préférence de 5 à 25 poises. (0,5 à 2,5 Pa.s), ladite viscosité étant mesurée à la température ambiante (environ 25°C) avec un Rhéomat 180 à l'aide d'un mobile 2, 3, 4 ou 5 selon la gamme de viscosité, à 200 s⁻¹.

La composition de l'invention peut être une dispersion aqueuse, un gel aqueux ou hydroalcoolique, contenant ou non une huile, ainsi qu'une émulsion eau-dans-huile ou huile-dans-eau.

Les polymères conformes à l'invention sont des polymères amphiphiles comportant au moins un monomère à insaturation éthylènique à groupement sulfonique, sous forme libre ou partiellement ou totalement neutralisée et comprenant au moins une partie hydrophobe.
On entend par polymère amphiphile, tout polymère comportant à la fois une partie hydrophile et une partie hydrophobe et notamment une chaîne grasse.

La partie hydrophobe présente dans les polymères de l'invention comporte de préférence de 6 à 50 atomes de carbone, plus préférentiellement de 6 à 22 atomes de carbone, plus préférentiellement encore de 6 à 18 atomes de carbone, et plus particulièrement de 12 à 18 atomes de carbone.

De façon préférentielle, les polymères conformes à l'invention sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Les polymères amphiphiles conformes à l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Les polymères amphiphiles selon l'invention peuvent être réticulés ou non-réticulés.
De préférence, on choisit des polymères amphiphiles réticulés.
Lorsqu'ils sont réticulés, les agents de réticulation peuvent être choisis parmi les composés à polyinsaturation oléfinique couramment utilisés pour la réticulation des polymères obtenus par polymérisation radicalaire.
On peut citer par exemple, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

On utilisera plus particulièrement le méthylène-bis-acrylamide, le méthacrylate d'allyle ou le triméthylol propane triacrylate (TMPTA). Le taux de réticulation variera en général de 0,01 à 10% en mole et plus particulièrement de 0,2 à 2% en mole par rapport au polymère.

on utilisera l'acide 2-acrylamido-2-méthylpropane-sulfonique (AMPS) ainsi que ses formes partiellement ou totalement neutralisées, comme monomère à groupement sulfonique.

Les polymères de l'invention sont choisis parmi les copolymères amphiphiles d'AMPS et d'au moins un monomère hydrophobe à insaturation éthylénique comportant au moins une partie hydrophobe ayant de 6 à 50 atomes de carbone et plus préférentiellement de 6 à 22 atomes de carbone et encore plus préférentiellement de 6 à 18 atomes de carbone et plus particulièrement 12 à 18 atomes de carbone.
Ces mêmes copolymères peuvent contenir en outre un ou plusieurs monomères éthyléniquement insaturés ne comportant pas de chaîne grasse tels que les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Les monomères hydrophobes à insaturation éthylénique de ces copolymères particuliers sont choisis parmi les acrylates ou les acrylamides de formule (I) suivante : 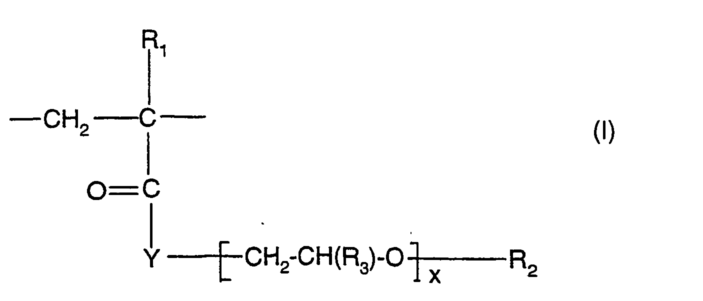 dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné hydrophobe comportant au moins de 6 à 50 atomes de carbone et plus préférentiellement de 6 à 22 atomes de carbone et encore plus préférentiellement de 6 à 18 atomes de carbone et plus particulièrement de 12 à 18 atomes de carbone ; x désigne un nombre de moles d'oxyde d'alkylène et varie de 3 à 100.

Le radical R₂ est choisi de préférence parmi les radicaux alkyles en C₆-C₁₈ linéaires (par exemple n-hexyle, n-octyle, n-décyle, n-hexadécyle, n-dodécyle), ramifiés ou cycliques (par exemple cyclododécane (C₁₂) ou adamantane (C₁₀)) ; les radicaux alkylperfluorés en C₆-C₁₈ (par exemple le groupement de formule -(CH₂)₂-(CF₂)₉-CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie de 3 à 100 et plus préférentiellement de 3 à 50 et encore plus préférentiellement de 7 à 25.

On citera plus particulièrement les copolymères constitués de motifs acide 2-acrylamido-2-méthylpropane-sulfonique (AMPS) de formule (II) suivante : 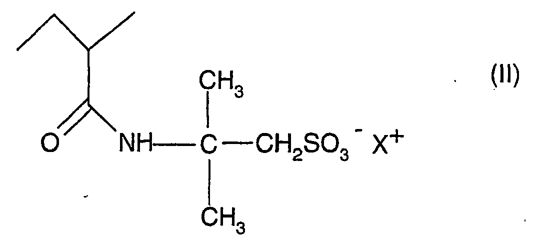 dans laquelle X⁺ est un proton, un cation de métal alcalin, un cation alcalino-terreux ou l'ion ammonium,
et de motifs de formule (III) suivante : 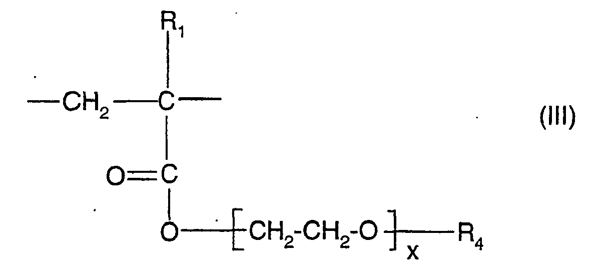 dans laquelle x désigne un nombre entier variant de 3 à 100, de préférence de 5 à 80 et plus préférentiellement de 7 à 25 ; R₁ a la même signification que celle indiquée ci-dessus dans la formule (I) et R₄ désigne un alkyle linéaire ou ramifié en C₆-C₂₂ et plus préférentiellement en C₁₀-C₂₂.

Les polymères particulièrement préférés sont ceux pour lesquels x = 25 , R₁ désigne méthyle et R₄ représente n-dodécyle ; ils sont décrits dans les articles de Morishima mentionnés ci-dessus.
Les polymères pour lesquels X⁺ désigne sodium ou ammonium sont plus particulièrement préférés.

Les polymères amphiphiles préférés conformes à l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le ABAH (2,2-azobis-[2-amidinopropane] hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ils sont notamment obtenus par polymérisation radicalaire en milieu tert-butanol dans lequel ils précipitent.
En utilisant la polymérisation par précipitation dans le tert-butanol, il est possible d'obtenir une distribution de la taille des particules du polymère particulièrement favorable pour ses utilisations.
La distribution de la taille des particules du polymère peut être déterminée par exemple par diffraction laser ou analyse d'image.
Une distribution intéressante pour de type de polymère et déterminée par analyse d'image est la suivante: 60,2% inférieur à 423 microns, 52,0% inférieur à 212 microns, 26,6% inférieur à 106 microns, 2,6% inférieur à 45 microns et 26,6% supérieur à 850 microns.
La réaction peut être conduite à une température comprise entre 0 et 150°C, de préférence entre 10 et 100°C , soit à pression atmosphérique, soit sous pression réduite. Elle peut aussi être réalisée sous atmosphère inerte, et de préférence sous azote.
Selon ce procédé, on a notamment polymérisé l'acide 2-acrylamido-2-méthylpropane-sulfonique (AMPS) ou l'un de ses sels de sodium ou d'ammonium, avec un ester de l'acide (méth)acrylique et,
- d'un alcool en C₁₀-C₁₈ oxyéthyléné par 8 moles d'oxyde d'éthylène (GENAPOL^{®} C-080 de la société HOECHST/CLARIANT),
- d'un alcool oxo en C₁₁ oxyéthyléné par 8 moles d'oxyde d'éthylène (GENAPOL^{®} UD-080 de la société HOECHST/CLARIANT),
- d'un alcool oxo en C₁₁ oxyéthyléné par 7 moles d'oxyde d'éthylène (GENAPOL^{®} UD-070 de la société HOECHST/CLARIANT),
- d'un alcool en C₁₂-C₁₄ oxyéthyléné par 7 moles d'oxyde d'éthylène (GENAPOL^{®} LA-070 de la société HOECHST/CLARIANT),
- d'un alcool en C₁₂-C₁₄ oxyéthyléné par 9 moles d'oxyde d'éthylène (GENAPOL^{®} LA-090 de la société HOECHST/CLARIANT),
- d'un alcool en C₁₂-C₁₄ oxyéthyléné par 11 moles d'oxyde d'éthylène (GENAPOL^{®} LA-110 de la société HOECHST/CLARIANT),
- d'un alcool en C₁₆-C₁₈ oxyéthyléné par 8 moles d'oxyde d'éthylène (GENAPOL^{®} T-080 de la société HOECHST/CLARIANT),
- d'un alcool en C₁₆-C₁₈ oxyéthyléné par 15 moles d'oxyde d'éthylène (GENAPOL^{®} T-150 de la société HOECHST/CLARIANT),
- d'un alcool en C₁₆-C₁₈ oxyéthyléné par 11 moles d'oxyde d'éthylène (GENAPOL^{®} T-110 de la société HOECHST/CLARIANT),
- d'un alcool en C₁₆-C₁₈ oxyéthyléné par 20 moles d'oxyde d'éthylène (GENAPOL^{®} T-200 de la société HOECHST/CLARIANT),
- d'un alcool en C₁₆-C₁₈ oxyéthyléné par 25 moles d'oxyde d'éthylène (GENAPOL^{®} T-250 de la société HOECHST/CLARIANT),
- d'un alcool en C₁₈-C₂₂ oxyéthyléné par 25 moles d'oxyde d'éthylène et/ou d'un alcool iso C₁₆-C₁₈ oxyéthyléné par 25 moles d'oxyde d'éthylène.

La concentration molaire en % des motifs de formule (II) et des motifs de formule (III) dans les polymères selon l'invention variera en fonction de l'application cosmétique souhaitée et des propriétés rhéologiques de la formulation recherchées. Elle peut varier entre 0,1 et 99,9% en moles.
De préférence pour les polymères les plus hydrophobes, la proportion molaire en motifs de formule (I) ou (III) varie de 50,1 à 99,9%, plus particulièrement de 70 à 95% et encore plus particulièrement de 80 à 90%.
De préférence pour les polymères peu hydrophobes, la proportion molaire en motifs de formule (I) ou (III) varie de 0,1 à 50%, plus particulièrement de 5 à 25% et encore plus particulièrement de 10 à 20%.
La distribution des monomères dans les polymères de l'invention peut être, par exemple, alternée, bloc (y compris multibloc) ou quelconque.
Selon l'invention, on préfère que les polymères aient des chaînes pendantes sensibles à la chaleur et dont la solution aqueuse présente une viscosité qui, au delà d'une certaine température seuil, augmente, ou demeure pratiquement constante quand la température augmente.
Plus particulièrement encore, on préfère les polymères dont la solution aqueuse présente une viscosité qui est faible en dessous d'une première température seuil et qui, au dessus de cette première température seuil croît vers un maximum quand la température augmente, et qui, au dessus d'une seconde température seuil décroît à nouveau quand la température augmente. Dans cet optique, on préfère que la viscosité des solutions de polymère en dessous de la première température seuil soit de 5 à 50%, en particulier de 10 à 30% de la viscosité maximum à la seconde température seuil.

Ces polymères, de préférence, conduisent dans l'eau à un phénomène de démixion par chauffage se traduisant par des courbes présentant, en fonction de la température et de la concentration, un minimum appelé LCST (Lower Critical Solution Temperature).

Les viscosités (mesurées à 25°C au viscosimètre Brookfield aiguille 7) des solutions aqueuses à 1% vont de préférence de 20 000 mPa.s à 100 000 mPa.s et plus particulièrement de 60 000 mPa.s à 70 000 mPa.s.

Les polymères amphiphiles conformes à l'invention sont présents dans les compositions dans des concentrations allant de 0,01 à 30% en poids, plus préférentiellement de 0,1 à 10%, encore plus préférentiellement de 0,1 à 5% en poids et plus particulièrement encore de 0,5 à 2% en poids.

La composition de l'invention comprend un composé insoluble dans l'eau tel que défini ci-dessus, généralement présent à raison de 0,001 à 40 % du poids total de la composition, de préférence de 0,05 à 25 % en poids.

De préférence, la concentration totale des composés de familles III et IV étant inférieure à 9% en poids par rapport au poids total de la composition.
Le rapport des composés de la famille IV) sur la totalité des corps gras de la composition est de préférence inférieure à 1.

Les composés insolubles dans l'eau sont particulièrement des agents conditionneurs des matières kératiniques. (Dans le cadre de la présente demande, on entend par agent conditionneur tout agent ayant pour fonction l'amélioration des propriétés cosmétiques des matières kératiniques telles que les cheveux, en particulier la douceur, le démêlage, le toucher, l'électricité statique).

Les composés insolubles conformément à l'invention peuvent être solides, liquides ou pâteux à température ambiante (25°C) et à pression atmosphérique , elles peuvent notamment se présenter sous forme d'huiles, de cires, de résines ou de gommes.

Les composés insolubles sont notamment dispersés dans les compositions sous forme de particules ayant généralement une taille moyenne comprise entre 2 nanomètres et 100 microns.

Les composés insolubles dans l'eau sont insolubles dans l'eau à une concentration supérieure ou égale à 0,1% en poids dans l'eau à 25°C, c'est à dire qu'ils ne forment pas une solution isotrope transparente.
I) Les huiles de synthèse sont notamment les polyoléfines en particulier les poly-α-oléfines et plus particulièrement :
   - de type polybutène, hydrogéné ou non, et de préférence polyisobutène, hydrogéné ou non.
      On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.
      A titre d'exemples de poly-α-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
   - de type polydécène, hydrogéné ou non.
      De tels produits sont vendus par exemple sous les dénominations ETHYLFLO par la société ETHYL CORP., et d'ARLAMOL PAO par la société ICI.
II) Les cires végétales ou synthétiques sont généralement solides à température ambiante (20°-25°C). Elles sont insolubles dans l'eau, solubles dans les huiles et sont capables de former un film hydrofuge.
   Sur la définition des cires, on peut citer par exemple P.D. Dorgan, Drug and Cosmetic Industry, Decembre 1983, pp. 30-33.
   La cire ou les cires végétales sont choisies parmi la cire de Carnauba, la cire de Candelila, la cire d'olivier, la cire de riz, la cire de jojoba hydrogénée ou les cires absolues de fleurs telles que la cire essentielle de fleur de cassis vendue par la Société BERTIN (France), la cerabellina ; les cires marines telles que celle vendue par la Société SOPHIM sous la référence M82.
III) les huiles essentielles naturelles ou synthétiques sont par exemple choisies parmi les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;
IV) Parmi les esters selon l'invention, on peut citer le lactate de cétyle ; le lactate d'alkyle en C₁₂-C₁₅ ; le lactate d'isostéaryle ; le lactate de lauryle ; le lactate de linoléyle ; le lactate d'oléyle ; les palmitates d'éthyle et d'isopropyle, les myristates d'alkyles en C1-C5 tels que le myristate d'isopropyle, de butyle, le stéarate d'isobutyle ; le malate de dioctyle.
   On peut aussi citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate le dicaprate de propylène glycol ; le citrate de triisopropyle ; le citrate de triisotéaryle ; trilactate de glycéryle ; le citrate de trioctyldodécyle ; le citrate de trioléyle.
   Parmi les esters cités ci-dessus, on préfère utiliser les palmitates d'éthyle et d'isopropyle, les myristates d'alkyles tels que le myristate d'isopropyle, de butyle, le stéarate de butyle, le stéarate d'isobutyle ; le malate de dioctyle.
   De préférence, la concentration en esters selon l'invention est comprise entre 0,2 et 15% en poids par rapport au poids total de la composition.
V) Les pigments non filtrants peuvent être blancs ou colorés, minéraux et/ou organiques, enrobés ou non, insolubles dans la phase grasse liquide. Ils sont destinés à colorer et/ou opacifier la composition. On peut citer, parmi les pigments minéraux, le dioxyde de titane, éventuellement traité en surface, les oxydes de zirconium ou de cérium, ainsi que les oxydes de zinc, de fer ou de chrome, le violet de manganèse, le bleu outremer et le bleu ferrique. Parmi les pigments organiques, on peut citer le noir de carbone, et les laques à base de carmin de cochenille, de baryum, strontium, calcium, aluminium. Ils Les pigments peuvent être présents dans la composition à raison de 0,05 à 40 % du poids de la composition finale, et de préférence à raison de 2 à 20 % pour une composition non pulvérulente. Pour une composition pulvérulente, il peuvent représenter jusqu'à 70 % du poids total de la composition.
   Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité. Les nacres peuvent être présentes dans la composition à raison de 0,05 à 20 % du poids total de la composition, de préférence à un taux de l'ordre de 1 à 15 %.
   Les charges peuvent être minérales ou organiques, lamellaires ou sphériques. On peut citer le talc, le mica, la silice, le kaolin, les poudres de Nylon^{®} (Orgasol^{®} notamment vendu par Atochem), de poly-β-alanine et de polyéthylène, le Téflon^{®}, la lauroyi-lysine, l'amidon, le nitrure de bore, les poudres de polymères de tétrafluoroéthylène, les microsphères creuses telles que l'Expancel^{®} (Nobel Industrie), le Polytrap^{®} (Dow Corning) et les microbilles de résine de silicone (Tospearl^{®} de Toshiba, par exemple), le carbonate de calcium précipité, le carbonate et l'hydro-carbonate de magnésium, les microsphères de silice creuses (Silica Beads de Maprecos), les microcapsules de verre
   ou de céramique. Les charges sont de préférence présentes à raison de 0,1 à 35 % du poids total de la composition, de préférence 0,5 à 15 %.
   Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine. Ils peuvent représenter de 0,01 à 20 % du poids de la compositions et mieux de 0,1 à 6 %.
VI) Les composés de type céramide utilisables selon la présente invention répondent préférentiellement à la formule générale (IV): 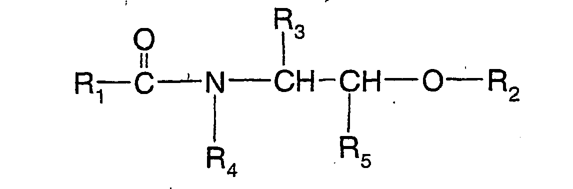
dans laquelle :
- R₁ désigne :
   - soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C₃₅ , le ou les hydroxyles du radical R₇ pouvant être estérifié par un acide gras saturé ou insaturé, linéaire ou ramifié, éventuellement mono ou polyhydroxylé, en C₁-C₃₅;
   - soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent.
   - soit un radical R₈-O-CO-(CH2)ₚ, R₈ désigne un radical hydrocarboné en C₁-C₂₀, p est un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄; de préférence, R₃ désigne un radical α-hydroxyalkyle en C₁₅-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH2)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium, sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

Parmi les composés de formule (IV), on préfère les céramides et/ou glycocéramides dont la structure est décrite par DOWNING dans Journal of Lipid Research Vol. 35, 2060-2068, 1994, ou ceux décrits dans la demande de brevet français FR-2 673 179, dont les enseignements sont ici inclus à titre de référence.

Les composés de type céramide plus particulièrement préférés selon l'invention sont les composés de formule (IV) pour lesquels R₁ désigne un alkyle saturé ou insaturé dérivé d'acides gras en C₁₄-C₂₂ éventuellement hydroxylé; R₂ désigne un atome d'hydrogène ; et R₃ désigne un radical linéaire en C₁₁₋₁₇ éventuellement hydroxylé et de préférence en C₁₃₋₁₅.

De tels composés sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
ou les mélanges de ces composés.

On peut aussi utiliser des mélanges spécifiques tels que par exemple les mélanges de céramide(s) 2 et de céramide(s) 5 selon la classification de DOWNING.

On peut également utiliser les composés de formule (IV) pour lesquels R₁ désigne un radical alkyle saturé ou insaturé dérivé d'acides gras en C₁₂-C₂₂ ; R₂ désigne un radical galactosyle ou sulfogalactosyle ; et R₃ désigne un radical hydrocarboné en C₁₂-C₂₂, saturé ou insaturé et de préférence un groupement -CH=CH-(CH₂)₁₂-CH₃.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés de formule (IV) décrits dans les demandes de brevet EP-A-0227994, EP-A-0 647 617, EP-A-0 736 522 et WO 94/07844.

De tels composés sont par exemple le QUESTAMIDE H (bis-(N-hydroxyéthyl N-cétyl) malonamide) vendu par la société QUEST, le N-(2-hydroxyéthyl)-N-(3-cétyloxy-2-hydroxypropyl)amide d'acide cétylique .

On peut également utiliser le N-docosanoyl N-méthyl-D-glucamine décrit dans la demande de brevet WO94/24097.

Des composés de type céramides particulièrement préférés selon l'invention sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique
- le N-docosanoyl N-méthyl-D-glucamine
ou les mélanges de ces composés.

La concentration en composés de type céramide peut varier entre 0,0001% et 20% en poids environ par rapport au poids total de la composition, et de préférence entre 0,001 et 10% environ et encore plus préférentiellement entre 0,005 et 3 % en poids.

Le milieu physiologiquement acceptable de la composition de l'invention comprend de préférence de l'eau. La quantité d'eau peut aller de 30 à 99,94 %, en poids et de préférence de 30 à 95 % en poids par rapport au poids total de la composition. Ce milieu peut contenir, outre l'eau, un ou plusieurs solvants organiques choisis dans le groupe constitué par les solvants organiques hydrophiles, les solvants organiques lipophiles, les solvants amphiphiles ou leurs mélanges. Les solvants sont choisis parmi les alcools inférieurs comportant de 1 à 8 atomes de carbone, tels que l'éthanol ; les polyols tels que la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose, le sucrose ; et leurs mélanges. La quantité de solvant(s) dans la composition de l'invention peut aller de 0,5 à 30 % en poids et de préférence de 5 à 20 % en poids par rapport au poids total de la composition.

La composition de l'invention a de préférence un pH compatible avec les matières kératiniques, c'est-à-dire allant de préférence de 3 à 10 et mieux de 4 à 7.

La composition de l'invention peut contenir également un ou plusieurs des additifs habituels dans le domaine cosmétique et/ou dermatologique, les actifs, les conservateurs, les gélifiants, les plastifiants, les antioxydants, les parfums, les absorbeurs d'odeur, les agents antimousse, les séquestrants (EDTA), les ajusteurs de pH acides ou basiques ou des tampons, les corps gras comme les huiles ou les cires autres que celles de l'invention, et leurs mélanges, dans la mesure où l'additif n'altère pas les propriétés recherchées pour la composition de l'invention. Les quantités de ces différents additifs sont celles classiquement utilisées dans les domaines considérés, et par exemple de 0,01 à 40 % du poids total de la composition.

La composition selon l'invention peut se présenter sous toutes les formes galéniques appropriées pour une application topique, et notamment sous forme de gel aqueux ou hydroalcoolique, d'émulsion eau-dans-huile (E/H) ou huile-dans-eau (H/E) ou multiple (E/H/E ou H/E/H), ou de dispersion aqueuse à base de vésicules lipidiques ioniques et/ou non-ioniques, contenant ou non une huile dispersée. Elle peut constituer par exemple un sérum, une crème ou un lait, une lotion épaissie ou une mousse.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse.

Lorsque la composition de l'invention est une émulsion, la proportion de la phase grasse (ou phase huileuse) peut aller de 5 à 80 % en poids, et de préférence de 10 à 50 % en poids par rapport au poids total de la composition et la proportion de phase aqueuse peut aller de 20 à 95 % et de préférence de 50 à 90 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsion peut éventuellement être préparée sans addition d'émulsionnants, grâce au caractère amphiphile du polymère. Quand l'émulsion contient un émulsionnant, celui-ci et, le coémulsionnant éventuellement présent sont présents en une proportion allant de 0,1 à 30 % en poids, et de préférence de 0,3 à 20 % en poids par rapport au poids total de la composition. L'émulsion peut, en outre, contenir des vésicules lipidiques.

Les autres corps gras additionnels pouvant être présents dans la phase huileuse sont par exemple les acides gras comportant de 8 à 30 atomes de carbone, comme l'acide stéarique, l'acide laurique, l'acide palmitique ; les cires comme la lanoline, la cire d'abeille, les cires de paraffine.

La composition selon l'invention peut être utilisée pour le traitement et le soin de la peau du visage et/ou du corps, des muqueuses (lèvres), du cuir chevelu et/ou des cheveux, et aussi pour le traitement de la peau sensible et/ou du cuir chevelu sensible.

Aussi, la présente invention a pour objet l'utilisation cosmétique de la composition cosmétique telle que définie ci-dessus, pour le traitement et le soin de la peau du visage et/ou du corps, des muqueuses, du cuir chevelu et/ou des cheveux.

La présente invention a encore pour objet l'utilisation de la composition telle que définie ci-dessus pour la fabrication d'une composition destinée à traiter la peau sensible et/ou le cuir chevelu sensible. Pour de plus amples détails concernant la peau sensible, on peut se référer au document EP-A-680749.

La composition selon l'invention peut constituer par exemple une crème de soin du visage, un lait corporel, un gel de douche, un gel de bain, une composition de coloration des cheveux, une composition de déformation permanente des cheveux, une composition de nettoyage et/ou de démaquillage du visage, une composition de protection solaire. Les compositions selon l'invention peuvent être également des compositions de shampooing, d'après-shampooing à rincer ou non.
Les compositions de l'invention peuvent également se présenter sous forme de compositions à rincer, à appliquer avant ou après une coloration, une décoloration, une permanente ou un défrisage ou encore entre les deux étapes d'une permanente ou d'un défrisage.

La présente invention a aussi pour objet un procédé de traitement cosmétique de la peau, du cuir chevelu, des cheveux, et/ou des muqueuses, caractérisé par le fait que l'on applique sur la peau, le cuir chevelu, les cheveux, et/ou les muqueuses, une composition telle que définie ci-dessus.

Les exemples ci-après de compositions selon l'invention, sont donnés à titre d'illustration et sans caractère limitatif. Les quantités y sont données en % en poids sauf mention contraire.

### EXEMPLES DE PREPARATION:

Préparation des esters (méth)acryliques éthoxylés.

Ils peuvent être notamment obtenus par action de (méth)acrylate de glycidyle, ou d'acide (méth)acrylique, ou d'un (méth)acrylate d'alkyle, ou d'un halogénure de (méth)acryloyle sur un alcool gras éthoxylé. On peut citer, à titre d'exemples non limitatifs, les préparations suivantes :
a) à partir du méthacrylate de glycidyle et du GENAPOL T-250
b) à partir de l'acide (méth)acrylique et du GENAPOL UD-070
c) à partir du (méth)acrylate de méthyle et du GENAPOL LA-090
d) à partir du chlorure de (méth)acryloyle et du GENAPOL UD-070.

a) Dans un réacteur tricol d'un litre équipé d'un agitateur, d'un thermomètre et d'un condensateur à reflux, on introduit 500g de Genapol T-250 et 75g de méthacrylate de glycidyle. On chauffe le mélange réactionnel à la température de 100°C pendant 2 heures, et on élimine l'excès de méthacrylate de glycidyle par distillation sous pression réduite. Le monomère obtenu peut être utilisé pour la polymérisation sans purification ultérieure.
b) Dans un réacteur tricol d'un litre équipé d'un agitateur, d'un thermomètre et d'un condensateur à reflux, on introduit 500g de Genapol UD-070, 100g d'acide (méth)acrylique et de l'acide p-toluènesulfonique comme catalyseur. On chauffe le mélange réactionnel au reflux pendant 2 heures, et l'on sépare l'excès d'acide et d'eau formée au cours de la réaction par distillation sous pression réduite. Le monomère obtenu peut être utilisé pour la polymérisation sans purification ultérieure.
c) Dans un réacteur tricol d'un litre équipé d'un agitateur, d'un thermomètre et d'un condensateur à reflux, on introduit 500g de Genapol LA-090, 100g de (méth)acrylate de méthyle et 20g de tétraisopropoxyde de titane. On chauffe le mélange réactionnel au reflux pendant 2 heures, et après séparation par distillation de l'alcool formé, on distille l'ester qui reste sous pression réduite.
   Le monomère obtenu peut être utilisé pour la polymérisation sans purification ultérieure.
d) Dans un réacteur tricol d'un litre équipé d'un agitateur, d'un thermomètre et d'un condensateur à reflux, on introduit 500g de Genapol UD-070, 110g de chlorure de (méth)acryloyle et 50g de carbonate de sodium. On chauffe le mélange réactionnel au reflux pendant 2 heures, et l'on sépare l'excès de chlorure d'acide par distillation sous pression réduite. Le monomère obtenu peut être utilisé pour la polymérisation sans purification ultérieure.

### Polymérisation selon la méthode de précipitation dans le tert-butanol

Dans un réacteur de 2 litres muni d'un condensateur à reflux, d'une prise de gaz, d'un thermomètre et d'un agitateur, on introduit 500ml de tert-butanol, et la quantité calculée d'AMPS. On neutralise le mélange en introduisant du NH3, et on ajoute le monomère préparé ci-dessus au mélange réactionnel. On rend le mélange réactionnel inerte par passage d'azote ou d'argon et lorsque la température intérieure a atteint 60°C, on introduit l'initiateur (AIBN) pour amorcer la polymérisation.
Après quelques minutes, le polymère ainsi préparé précipite. On porte le mélange à reflux pendant 2 heures, et on sépare le polymère du solvant par filtration à la trompe, puis on le sèche sous pression réduite.
De la façon décrite ci-dessus, on a préparé les polymères suivants :
(à partir des réactants suivants en des quantités exprimées en grammes)

| | | | | |
|---|---|---|---|---|
| Méthacrylate de Genapol T-250 | 10 | 20 | 30 | 97 |
| AMPS neutralisé par NH3 ..... | 90 | 80 | 90 | 3 |
| Méthylène-bis-acrylamide (réticulant).. | | | 1,5 | |
| Méthacrylate d'allyle (réticulant)......... | | 1,7 | | |
| TMPTA (réticulant)........................ | 1,8 | | | 1,8 |
| Azobisisobutyronitrile (initiateur)...... | | | 1 | |
| Peroxyde dilauryle (initiateur)......... | 1 | 1 | | 1 |
| Tert-butanol..... | 300 | 300 | 300 | 300 |

### Exemple de formulation :

On a préparé une composition d'après-shampooing à rincer :

| | |
|---|---|
| - Copolymère réticulé par du méthylène-bis-acrylamide constitué de 75% en poids de motifs AMPS neutralisés par NH₃ et de 25% en poids de motifs de formule (III) dans laquelle R₁=H, R₄=C₁₆-C₁₈ et x=25 | 0,75 g |
| - Myristate d'isopropyle | 1 g |
| - Eau déminéralisée | qsp 100 g |

La composition se répartit facilement sur la chevelure. Elle se rince bien et les cheveux sont doux et se démêlent bien.

Des résultats similaires ont été obtenus en remplaçant le copolymère acide acrylamido-2-méthyl-2-propane-sulfonique/n-dodécylacrylamide neutralisé par la soude de l'exemple ci-dessus par un copolymère réticulé par du méthacrylate d'allyle constitué de 90% en poids de motifs AMPS neutralisés par NH₃ et de 10% en poids de motifs méthacrylate de GENAPOL T-250 [motifs de formule (III) dans, laquelle R₁=CH₃, R₄=C₁₆-C₁₈ et x=25], ou par un copolymère réticulé par du méthacrylate d'allyle constitué de 80% en poids de motifs AMPS neutralisés par NH₃ et de 20% en poids de motifs méthacrylate de GENAPOL T-250 [motifs de formule (III) dans laquelle R₁=CH₃, R₄=C₁₆-C₁₈ et x=25].

## Revendications

1. Composition pour application topique, comportant une phase aqueuse comprenant au moins un polymère amphiphile choisis parmi les copolymères amphiphiles de 2-acrylamido-2-méthylpropane-sulfonique (AMPS) sous forme libre ou partiellement ou totalement neutralisée et d'au moins un monomère hydrophobe à insaturation éthylénique comportant au moins une partie hydrophobe choisi parmi les acrylates ou les acrylamides de formule (I) suivante : 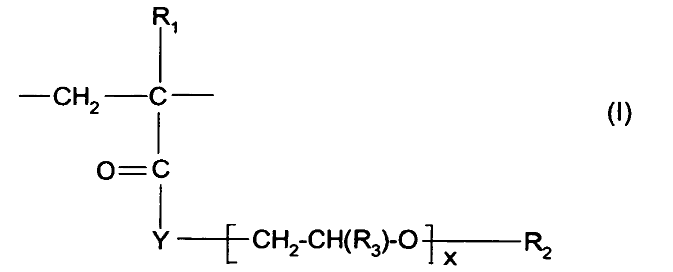 dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné hydrophobe comportant au moins de 6 à 50 atomes de carbone et plus préférentiellement de 6 à 22 atomes de carbone et encore plus préférentiellement de 6 à 18 atomes de carbone et plus particulièrement de 12 à 18 atomes de carbone ; x désigne un nombre de moles d'oxyde d'alkylène et varie de 3 à 100,
et au moins un composé insoluble dans l'eau choisi parmi :
I) les huiles de synthèse
II) les cires végétales et les cires synthétiques choisies parmi les cires de polyéthylène, de polyoléfines ou de Fischer-Tropsch,
III) les huiles végétales choisies parmi les huiles de tournesol, de maïs, de soja, d'avocat, de courge, de pépins de raisin, de sésame, de noisette, l'huile de Purcellin, les huiles essentielles naturelles ou synthétiques
IV) les esters d'acides di, tri ou tétracarboxyliques ,les monoesters d'acides monocarboxyliques aliphatiques saturés ou insaturés, linéaires ou ramifiés en C₁-C₅ et d'alcools ou de polyols aliphatiques saturés ou insaturés, linéaires ou ramifiés en C₁-C₂₆, les monoesters d'acides monocarboxyliques aliphatiques saturés ou insaturés, linéaires ou ramifiés en C₁-C₂₆ et d'alcools aliphatiques saturés ou insaturés, linéaires ou ramifiés en C₁-C₅,
V) les pigments non filtrants, les nacres et les charges,
VI) les composés de type céramide répondant à la formule générale (IV): 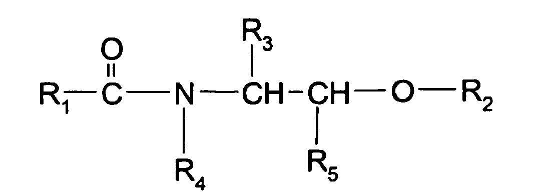
dans laquelle :
- R₁ désigne:
- soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C₃₅ , le ou les hydroxyles du radical R₇ pouvant être estérifié par un acide gras saturé ou insaturé, linéaire ou ramifié, éventuellement mono ou polyhydroxylé, en C₁-C₃₅;
- soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent.
- soit un radical R₈-O-CO-(CH2)ₚ, R₈ désigne un radical hydrocarboné en C₁-C₂₀, p est un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄ ; de préférence, R₃ désigne un radical α-hydroxyalkyle en C₁₅-C₂₆, le groupement hydroxyle étant éventuellement estérifié par un α-hydroxyacide en C₁₆-C₃₀;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH2)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle ;

2. Composition selon la revendication 1, **caractérisée par le fait que** la partie hydrophobe du polymère amphiphile comporte de 6 à 50 atomes de carbone.

3. Composition selon la revendication 2, **caractérisée par le fait que** la partie hydrophobe du polymère amphiphile comporte de 6 à 22 atomes de carbone.

4. Composition selon la revendication 3, **caractérisée par le fait que** la partie hydrophobe du polymère amphiphile comporte de 6 à 18 atomes de carbone.

5. Composition selon la revendication 4, **caractérisée par le fait que** la partie hydrophobe du polymère amphiphile comporte de 12 à 18 atomes de carbone.

6. Composition selon l'une quelconque des revendications 1 à 5, **caractérisée par le fait que** les polymères amphiphiles sont neutralisés partiellement ou totalement par une base minérale ou organique.

7. Composition selon l'une quelconque des revendications 1 à 6, **caractérisée par le fait que** les polymères amphiphiles ont un poids moléculaire moyen en nombre allant de 1000 à 20 000 000 g/mole.

8. Composition selon la revendication 7, **caractérisée par le fait que** le poids moléculaire moyen en nombre varie de 20 000 à 5 000 000 g/mole.

9. Composition selon la revendication 8, **caractérisée par le fait que** le poids moléculaire moyen en nombre varie de 100 000 à 1 500 000 g/mole.

10. Composition selon l'une quelconque des revendications précédentes, **caractérisée par le fait qu'**une solution aqueuse à 1% en poids desdits polymères présente à la température de 25°C une viscosité mesurée au viscosimètre Brookfield, aiguille 7, allant de 20 000 mPa.s à 100 000 mPa.s.

11. Composition selon l'une quelconque des revendications précédentes, **caractérisée par le fait que** les polymères amphiphiles sont préparés par polymérisation radicalaire par précipitation dans le tert-butanol.

12. Composition selon l'une quelconque des revendications précédentes, **caractérisée par le fait que** les polymères amphiphiles sont réticulés ou non-réticulés.

13. Composition selon la revendication 12, **caractérisée par le fait que** les polymères amphiphiles sont réticulés.

14. Composition selon la revendication 13, **caractérisée par le fait que** le ou les agents de réticulation sont choisis parmi les composés à polyinsaturation oléfinique.

15. Composition selon la revendication 14, **caractérisée par le fait que** le ou les agents de réticulation sont choisis parmi le méthylène-bis-acrylamide, le méthacrylate d'allyle ou le triméthylol propane triacrylate (TMPTA).

16. Composition selon l'une quelconque des revendications 13 à 15, **caractérisée par le fait que** le taux de réticulation varie de préférence de 0,01 à 10% en moles et plus particulièrement de 0,2 à 2% en moles par rapport au polymère.

17. Composition selon l'une des revendications 1 à 16, **caractérisée par le fait que** le radical hydrophobe R₂ de la formule (I) est choisi parmi les radicaux alkyles en C₆-C₁₈, linéaires, ramifiés ou cycliques ; les radicaux alkylperfluorés en C₆-C₁₈; le radical cholestéryle ou un ester de cholestérol ; les groupes polycycliques aromatiques.

18. Composition selon l'une des revendications 1 à 17, **caractérisée par le fait que** la chaîne polyoxyalkylénée est constituée de motifs oxyde d'éthylène et/ou de motifs oxyde de propylène.

19. Composition selon la revendication 18, **caractérisée par le fait que** la chaîne polyoxyalkylénée est constituée uniquement de motifs oxyde d'éthylène.

20. Composition selon l'une des revendications 1 à 19, **caractérisée par le fait que** le nombre de motifs oxyalkylénés varie de 3 à 50.

21. Composition selon la revendication 20, **caractérisée par le fait que** le nombre de motifs oxyalkylénés varie de 7 à 25.

22. Composition selon les revendications 1 à 21, **caractérisée par le fait que** le polymère amphiphile d'AMPS est choisi parmi les copolymères constitués de motifs acide 2-acrylamido-2-méthylpropane-sulfonique (AMPS) de formule (II) suivante : 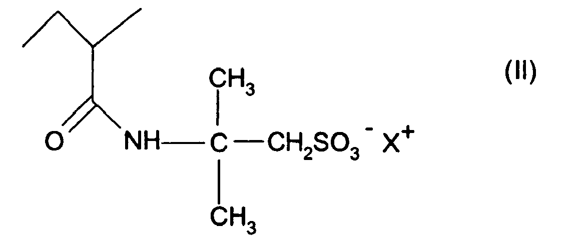 dans laquelle X⁺ est un proton, un cation de métal alcalin, un cation alcalino-terreux ou l'ion ammonium,
et de motifs de formule (III) suivante : 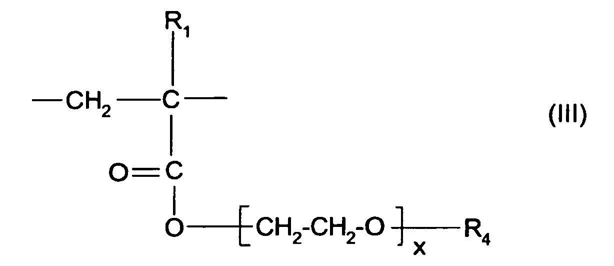 dans laquelle x désigne un nombre entier variant de 3 à 100, de préférence de 5 à 80 et plus préférentiellement de 7 à 25 ; R₁ a la même signification que celle indiquée ci-dessus dans la formule (I) et R₄ désigne un alkyle linéaire ou ramifié en C₆-C₂₂ et plus préférentiellement en C₁₀-C₂₂.

23. Composition selon la revendication 22, **caractérisée par le fait que** x = 25 , R₁ est méthyle et R₄ est n-dodécyle.

24. Composition selon les revendications 1 à 23, **caractérisée par le fait que** la proportion molaire en % des motifs de formule (I) ou des motifs de formule (III) dans les polymères varie de 50,1 à 99,9%.

25. Composition selon la revendications 1 à 23, **caractérisée par le fait que** la proportion molaire en % des motifs de formule (I) ou des motifs de formule (III) dans les polymères varie de 0,1 à 50%.

26. Composition selon l'une quelconque des revendications précédentes, **caractérisée par le fait que** les polymères amphiphiles sont présents dans des concentrations allant de 0,01 à 30% en poids, plus préférentiellement de 0,1 à 10% en poids, encore plus préférentiellement de 0,1 à 5% en poids, et plus particulièrement encore de 0,5 à 2% en poids par rapport au poids total de la composition.

27. Composition selon la revendication précédente, **caractérisée par le fait que** les huiles de synthèse sont des polyoléfines de type polybutène, hydrogéné ou non, ou de type polydécène, hydrogéné ou non.

28. Composition selon l'une quelconque des revendications précédentes, **caractérisée par le fait que** les huiles essentielles naturelles ou synthétiques sont choisies parmi les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote.

29. Composition selon l'une quelconque des revendications précédentes, **caractérisée par le fait que** les cires végétales sont choisies parmi la cire de Carnauba, la cire de Candelila, la cire d'olivier, la cire de riz, la cire de jojoba hydrogénée ou les cires absolues de fleurs telles que la cire essentielle de fleur de cassis, les cires marines.

30. Composition selon l'une quelconque des revendications précédentes, **caractérisée par le fait que** les composés de type céramides sont choisis parmi:
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique .
- le N-docosanoyl N-méthyl-D-glucamine
ou les mélanges de ces composés.

31. Composition selon l'une quelconque des revendications précédentes, **caractérisée en ce que** les esters sont choisis parmi les palmitates d'éthyle et d'isopropyle , le myristate d'isopropyle, de butyle, le stéarate de butyle, le stéarate d'isobutyle, le malate de dioctyle.

32. Composition selon l'une quelconque des revendications précédentes, **caractérisée en ce que** les pigments sont choisis parmi le dioxyde de titane, éventuellement traité en surface, les oxydes de zirconium ou de cérium, ainsi que les oxydes de zinc, de fer ou de chrome, le violet de manganèse, le bleu outremer et le bleu ferrique, le noir de carbone, et les laques à base de carmin de cochenille, de baryum, strontium, calcium, aluminium, les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité.

33. Composition selon l'une quelconque des revendications précédentes, **caractérisée en ce que** les charges sont minérales ou organiques, lamellaires ou sphériques.

34. Composition selon la revendication précédente, **caractérisée en ce que** les charges sont choisies parmi le talc, le mica, la silice, le kaolin, les poudres de Nylon^{®}, de poly-β-alanine et de polyéthylène, le Téflon^{®}, la lauroyl-lysine, l'amidon, le nitrure de bore, les poudres de polymères de tétrafluoroéthylène, les microsphères creuses et les microbilles de résine de silicone, le carbonate de calcium précipité, le carbonate et l'hydro-carbonate de magnésium, les microsphères de silice creuses, les microcapsules de verre ou de céramique.

35. Composition selon l'une quelconque des revendications précédentes, **caractérisée en ce que** les composés insolubles dans l'eau sont présents à une concentration comprise entre 0,001 % et 40 % en poids par rapport au poids total de la composition.

36. Composition selon l'une quelconque des revendications précédentes, **caractérisée en ce que** le milieu physiologiquement acceptable est constitué d'eau ou d'eau et d'au moins un solvant organique choisi dans le groupe constitué par les solvants organiques hydrophiles, les solvants organiques lipophiles, les solvants amphiphiles ou leurs mélanges.

37. Composition selon l'une quelconque des revendications précédentes, **caractérisée en ce qu'**elle constitue une crème de soin du visage, un lait corporel, un gel de douche, un gel de bain, une composition de coloration des cheveux, une composition de déformation permanente des cheveux, une composition de nettoyage et/ou de démaquillage du visage, un shampooing, un après-shampooing.

38. Utilisation cosmétique de la composition selon l'une quelconque des revendications 1 à 37, pour le traitement et le soin de la peau du visage et/ou du corps, des muqueuses, du cuir chevelu et/ou des cheveux.

39. Utilisation de la composition selon l'une quelconque des revendications 1 à 37, pour la fabrication d'une composition destinée à traiter la peau sensible et/ou le cuir chevelu sensible.

40. Procédé de traitement cosmétique de la peau, du cuir chevelu, des cheveux, des ongles et/ou des muqueuses, **caractérisé par le fait que** l'on applique sur la peau, le cuir chevelu, les cheveux, les ongles et/ou les muqueuses, une composition selon l'une quelconque des revendications 1 à 37.

41. Utilisation d'un polymère amphiphile comportant au moins un monomère à insaturation éthylènique à groupement sulfonique, sous forme libre ou partiellement ou totalement neutralisée et comprenant au moins une partie hydrophobe tel que défini à l'une quelconque des revendications 1 à 26, pour stabiliser une composition cosmétique ou dermatologique contenant au moins un composé insoluble dans l'eau et/ou améliorer son dépôt.

## Claims

1. Composition for topical application, containing an aqueous phase comprising at least one amphiphilic polymer chosen from amphiphilic copolymers of 2-acrylamido-2-methylpropanesulfonic (AMPS) in free or partially or totally neutralized form and of at least one ethylenically unsaturated hydrophobic monomer containing at least one hydrophobic portion chosen from the acrylates and acrylamides of formula (I) below: 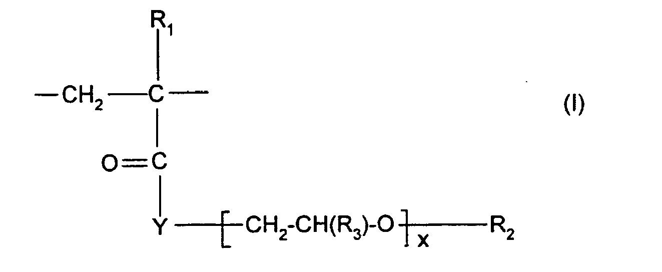 in which R₁ and R₃, which may be identical or different, denote a hydrogen atom or a linear or branched C₁-C₆ alkyl radical (preferably methyl); Y denotes O or NH; R₂ denotes a hydrophobic hydrocarbon-based radical containing at least from 6 to 50 carbon atoms, more preferentially from 6 to 22 carbon atoms, even more preferentially from 6 to 18 carbon atoms and more particularly from 12 to 18 carbon atoms; x denotes a number of moles of alkylene oxide and ranges from 3 to 100, and at least one water-insoluble compound chosen from:
I) synthetic oils,
II) plant waxes and synthetic waxes chosen from polyethylene waxes, polyolefin waxes and Fischer-Tropsch waxes,
III) plant oils chosen from sunflower oil, corn oil, soybean oil, avocado oil, marrow oil, grapeseed oil, sesame seed oil, hazelnut oil, purcellin oil and natural or synthetic essential oils,
IV) esters of di-, tri- or tetracarboxylic acids, monoesters of saturated or unsaturated, linear or branched C₁-C₅ aliphatic monocarboxylic acids and of saturated or unsaturated, linear or branched C₁-C₂₆ aliphatic alcohols or polyols, and monoesters of saturated or unsaturated, linear or branched C₁-C₂₆ aliphatic monocarboxylic acids and of saturated or unsaturated, linear or branched C₁-C₅ aliphatic alcohols,
V) non-screening pigments, nacres and fillers,
VI) compounds of ceramide type corresponding to the general formula (IV): 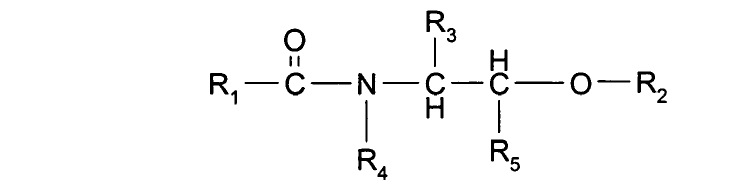
in which:
- R₁ denotes:
- either a saturated or unsaturated, linear or branched C₁-C₅₀, preferably C₅-C₅₀, hydrocarbon-based radical, this radical possibly being substituted with one or more hydroxyl groups optionally esterified with an acid R₇COOH, R₇ being a saturated or unsaturated, linear or branched, optionally mono- or polyhydroxylated C₁-C₃₅ hydrocarbon-based radical, the hydroxyl(s) of the radical R₇ possibly being esterified with a saturated or unsaturated, linear or branched, optionally mono- or polyhydroxylated C₁-C₃₅ fatty acid;
- or a radical R"-(NR-CO)-R', R denotes a hydrogen atom or a mono- or polyhydroxylated, preferentially monohydroxylated, C₁-C₂₀ hydrocarbon-based radical, R' and R" are hydrocarbon-based radicals, the sum of the carbon atoms of which is between 9 and 30, R' being a divalent radical;
- or a radical R₈-O-CO- (CH₂)ₚ, R₈ denotes a C₁-C₂₀ hydrocarbon-based radical, p is an integer ranging from 1 to 12;
- R₂ is chosen from a hydrogen atom, a radical of saccharide type, in particular a (glycosyl)ₙ, (galactosyl)ₘ or sulfogalactosyl radical, a sulfate or phosphate residue, a phosphorylethylamine radical and a phosphorylethylammonium radical, in which n is an integer ranging from 1 to 4 and m is an integer ranging from 1 to 8;
- R₃ denotes a hydrogen atom or a saturated or unsaturated, hydroxylated or non-hydroxylated C₁-C₃₃ hydrocarbon-based radical, the hydroxyl(s) possibly being esterified with a mineral acid or an acid R₇COOH, R₇ having the same meanings as above, the hydroxyl(s) possibly being etherified with a (glycosyl)ₙ, (galactosyl)ₘ, sulfogalactosyl, phosphorylethylamine or phosphorylethylammonium radical, R₃ also possibly being substituted with one or more C₁-C₁₄ alkyl radicals; preferably, R₃ denotes a C₁₅-C₂₆ α-hydroxyalkyl radical, the hydroxyl group being optionally esterified with a C₁₆-C₃₀ α-hydroxy acid;
- R₄ denotes a hydrogen atom, a methyl or ethyl radical, a saturated or unsaturated, linear or branched, optionally hydroxylated C₃-C₅₀ hydrocarbon-based radical or a radical -CH₂-CHOH-CH₂-O-R₆ in which R₆ denotes a C₁₀-C₂₆ hydrocarbon-based radical or a radical R₈-O-CO-(CH₂)ₚ, R₈ denotes a C₁-C₂₀ hydrocarbon-based radical, p is an integer ranging from 1 to 12;
- R₅ denotes a hydrogen atom or a saturated or unsaturated, linear or branched, optionally mono- or polyhydroxylated C₁-C₃₀ hydrocarbon-based radical, the hydroxyl(s) possibly being etherified with a (glycosyl)ₙ, (galactosyl)ₘ, sulfogalactosyl, phosphorylethylamine or phosphorylethylammonium radical, with the proviso that when R₃ and R₅ denote hydrogen or when R₃ denotes hydrogen and R₅ denotes methyl, then R₄ does not denote a hydrogen atom or a methyl or ethyl radical.

2. Composition according to Claim 1, **characterized in that** the hydrophobic portion of the amphiphilic polymer contains from 6 to 50 carbon atoms.

3. Composition according to Claim 2, **characterized in that** the hydrophobic portion of the amphiphilic polymer contains from 6 to 22 carbon atoms.

4. Composition according to Claim 3, **characterized in that** the hydrophobic portion of the amphiphilic polymer contains from 6 to 18 carbon atoms.

5. Composition according to Claim 4, **characterized in that** the hydrophobic portion of the amphiphilic polymer contains from 12 to 18 carbon atoms.

6. Composition according to any one of Claims 1 to 5, **characterized in that** the amphiphilic polymers are partially or totally neutralized with a mineral or organic base.

7. Composition according to any one of Claims 1 to 6, **characterized in that** the amphiphilic polymers have a number-average molecular weight ranging from 1000 to 20 000 000 g/mol.

8. Composition according to Claim 7, **characterized in that** the number-average molecular weight ranges from 20 000 to 5 000 000 g/mol.

9. Composition according to Claim 8, **characterized in that** the number-average molecular weight ranges from 100 000 to 1 500 000 g/mol.

10. Composition according to any one of the preceding claims, **characterized in that** an aqueous solution containing 1% by weight of the said polymers has, at a temperature of 25°C, a viscosity, measured using a Brookfield viscometer with a No. 7 needle, ranging from 20 000 mPa.s to 100 000 mPa.s.

11. Composition according to any one of the preceding claims, **characterized in that** the amphiphilic polymers are prepared by free-radical precipitation polymerization in tert-butanol.

12. Composition according to any one of the preceding claims, **characterized in that** the amphiphilic polymers are crosslinked or non-crosslinked.

13. Composition according to Claim 12, **characterized in that** the amphiphilic polymers are crosslinked.

14. Composition according to Claim 13, **characterized in that** the crosslinking agent(s) is (are) chosen from polyolefinically unsaturated compounds.

15. Composition according to Claim 14, **characterized in that** the crosslinking agent(s) is (are) chosen from methylenebisacrylamide, allyl methacrylate and trimethylolpropane triacrylate (TMPTA).

16. Composition according to any one of Claims 13 to 15, **characterized in that** the degree of crosslinking preferably ranges from 0.01 mol% to 10 mol% and more particularly from 0.2 mol% to 2 mol% relative to the polymer.

17. Composition according to one of Claims 1 to 16, **characterized in that** the hydrophobic radical R₂ of formula (I) is chosen from linear, branched or cyclic C₆-C₁₈ alkyl radicals; C₆-C₁₈ alkylperfluoro radicals; the cholesteryl radical or a cholesterol ester; aromatic polycyclic groups.

18. Composition according to one of Claims 1 to 17, **characterized in that** the polyoxyalkylene chain consists of ethylene oxide units and/or propylene oxide units.

19. Composition according to Claim 18, **characterized in that** the polyoxyalkylene chain consists solely of ethylene oxide units.

20. Composition according to one of Claims 1 to 19, **characterized in that** the number of oxyalkylene units ranges from 3 to 50.

21. Composition according to Claim 20, **characterized in that** the number of oxyalkylene units ranges from 7 to 25.

22. Composition according to Claims 1 to 21, **characterized in that** the amphiphilic polymer of AMPS is chosen from copolymers consisting of 2-acrylamido-2-methylpropanesulfonic acid (AMPS) units of formula (II) below: 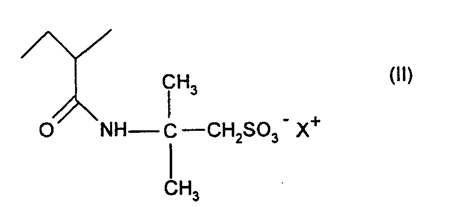 in which X⁺ is a proton, an alkali metal cation, an alkaline-earth metal cation or the ammonium ion,
and of units of formula (III) below: 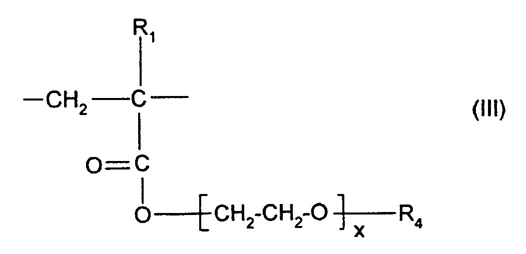 in which x denotes an integer ranging from 3 to 100, preferably from 5 to 80 and more preferentially from 7 to 25; R₁ has the same meaning as that given above in formula (I) and R₄ denotes a linear or branched C₆-C₂₂ and more preferentially C₁₀-C₂₂ alkyl.

23. Composition according to Claim 22, **characterized in that** x = 25, R₁ is methyl and R₄ is n-dodecyl.

24. Composition according to Claims 1 to 23, **characterized in that** the percentage molar proportion of units of formula (I) or of units of formula (III) in the polymers ranges from 50.1% to 99.9%.

25. Composition according to Claims 1 to 23, **characterized in that** the percentage molar proportion of units of formula (I) or of units of formula (III) in the polymers ranges from 0.1% to 50%.

26. Composition according to any one of the preceding claims, **characterized in that** the amphiphilic polymers are present in concentrations ranging from 0.01% to 30% by weight, more preferentially from 0.1% to 10% by weight, even more preferentially from 0.1% to 5% by weight and even more particularly from 0.5% to 2% by weight relative to the total weight of the composition.

27. Composition according to the preceding claim, **characterized in that** the synthetic oils are polyolefins of hydrogenated or non-hydrogenated polybutene type or of hydrogenated or non-hydrogenated polydecene type.

28. Composition according to any one of the preceding claims, **characterized in that** the natural or synthetic essential oils are chosen from eucalyptus oil, lavandin oil, lavender oil, vetiver oil, Litsea cubeba oil, lemon oil, sandalwood oil, rosemary oil, camomile oil, savory oil, nutmeg oil, cinnamon oil, hyssop oil, caraway oil, orange oil, geraniol oil, cade oil and bergamot oil.

29. Composition according to any one of the preceding claims, **characterized in that** the plant waxes are chosen from carnauba wax, candelilla wax, olive wax, rice wax, hydrogenated jojoba wax or the absolute waxes of flowers, such as the essential wax of blackcurrant blossom, and marine waxes.

30. Composition according to any one of the preceding claims, **characterized in that** the compounds of ceramide type are chosen from:
- 2-N-linoleoylaminooctadecane-1,3-diol,
- 2-N-oleoylaminooctadecane-1,3-diol,
- 2-N-palmitoylaminooctadecane-1,3-diol,
- 2-N-stearoylaminooctadecane-1,3-diol,
- 2-N-behenoylaminooctadecane-1,3-diol,
- 2-N-[2-hydroxypalmitoyl]aminooctadecane-1,3-diol,
- 2-N-stearoylaminooctadecane-1,3,4-triol and in particular N-stearoyl phytosphingosine,
- 2-N-palmitoylaminohexadecane-1,3-diol,
- bis(N-hydroxyethyl-N-cetyl)malonamide,
- cetylic acid N-(2-hydroxyethyl)-N-(3-cetyloxy-2-hydroxypropyl) amide,
- N-docosanoyl-N-methyl-D-glucamine,
or mixtures of these compounds.

31. Composition according to any one of the preceding claims, **characterized in that** the esters are chosen from ethyl palmitate, isopropyl palmitate, isopropyl myristate, butyl myristate, butyl stearate, isobutyl stearate and dioctyl malate.

32. Composition according to any one of the preceding claims, **characterized in that** the pigments are chosen from titanium dioxide, optionally surface-treated, zirconium oxide or cerium oxide, and also zinc oxide, iron oxide or chromium oxide, manganese violet, ultramarine blue and ferric blue, carbon black, and lakes based on cochineal carmine or on barium, strontium, calcium or aluminium, white nacreous pigments such as mica coated with titanium or with bismuth oxychloride, coloured nacreous pigments such as titanium mica with iron oxides, titanium mica especially with ferric blue or with chromium oxide, and titanium mica with an organic pigment of the abovementioned type.

33. Composition according to any one of the preceding claims, **characterized in that** the fillers are mineral or organic, and lamellar or spherical.

34. Composition according to the preceding claim, **characterized in that** the fillers are chosen from talc, mica, silica, kaolin, Nylon^{®} powder, poly-β-alanine powder and polyethylene powder, Teflon^{®}, lauroyllysine, starch, boron nitride, tetrafluoroethylene polymer powders, hollow microspheres and silicone resin microbeads, precipitated calcium carbonate, magnesium carbonate, magnesium hydrogen carbonate, hollow silica microspheres, and glass or ceramic microcapsules.

35. Composition according to any one of the preceding claims, **characterized in that** the water-insoluble compounds are present at a concentration of between 0.001% and 40% by weight relative to the total weight of the composition.

36. Composition according to any one of the preceding claims, **characterized in that** the physiologically acceptable medium consists of water or of water and at least one organic solvent chosen from the group consisting of hydrophilic organic solvents, lipophilic organic solvents and amphiphilic solvents, or mixtures thereof.

37. Composition according to any one of the preceding claims, **characterized in that** it is a facial care cream, a body milk, a shower gel, a bath gel, a hair colouring composition, a hair permanent-reshaping composition, a facial cleansing and/or makeup-removing composition, a shampoo or a hair conditioner.

38. Cosmetic use of the composition according to any one of Claims 1 to 37, for treating and caring for facial and/or bodily skin, mucous membranes, the scalp and/or the hair.

39. Use of the composition according to any one of Claims 1 to 37, for the manufacture of a composition for treating sensitive skin and/or a sensitive scalp.

40. Cosmetic process for treating the skin, the scalp, the hair, the nails and/or mucous membranes, **characterized in that** a composition according to any one of Claims 1 to 37 is applied to the skin, the scalp, the hair, the nails and/or mucous membranes.

41. Use of an amphiphilic polymer comprising at least one ethylenically unsaturated monomer containing a sulfonic group, in free or partially or totally neutralized form and comprising at least one hydrophobic portion as defined in any one of Claims 1 to 26, for stabilizing a cosmetic or dermatological composition containing at least one water-insoluble compound and/or for increasing its deposition.

## Patentansprüche

1. Zusammensetzung für die topische Anwendung, die eine wässrige Phase enthält, die mindestens ein amphiphiles Polymer, das unter den amphiphilen Copolymeren von in freier oder teilweise oder ganz neutralisierter Form vorliegender 2-Acrylamido-2-methylpropan-sulfonsäure (AMPS) und mindestens einem hydrophoben Monomer mit einer ethylenisch ungesättigten Bindung ausgewählt ist, das mindestens einen hydrophoben Teil aufweist und das unter den Acrylaten oder den Acrylamiden der folgenden Formel (I) ausgewählt ist: 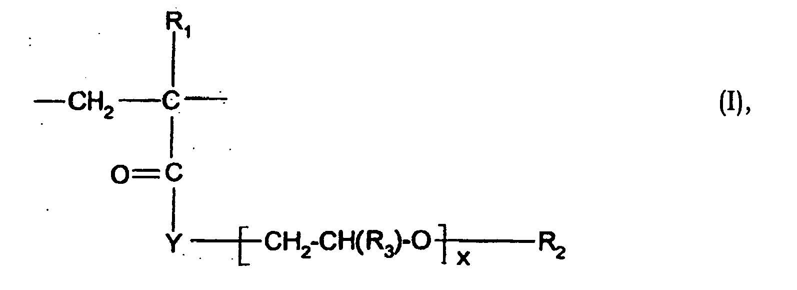 worin bedeuten:
R₁ und R₃, die gleich oder verschieden sind, Wasserstoff oder eine geradkettige oder verzweigte C₁₋₆-Alkylgruppe (vorzugsweise Methyl);
Y O oder NH;
R₂ eine hydrophobe Kohlenwasserstoffgruppe mit mindestens 6 bis 50 Kohlenstoffatomen, bevorzugter 6 bis 22 Kohlenstoffatomen, noch bevorzugter 6 bis 18 Kohlenstoffatomen und insbesondere 12 bis 18 Kohlenstoffatomen;
x eine Alkylenoxid-Molzahl, die im Bereich von 3 bis 100 liegt, und die mindestens eine in Wasser unlösliche Verbindung enthält, die ausgewählt ist unter:
I) synthetischen Ölen
II) pflanzlichen Wachsen und synthetischen Wachsen, die unter den Polyethylenwachsen, Polyolefinwachsen oder durch Fischer-Tropsch-Synthese hergestellten Wachsen ausgewählt sind,
III) pflanzlichen Ölen, die unter Sonnenblumenöl, Maisöl, Sojaöl, Avocadoöl, Kürbiskernöl, Traubenkernöl, Sesamöl, Haselnussöl, Purcellinöl, natürlichen oder synthetischen etherischen Ölen ausgewählt sind,
IV) Di-, Tri- oder Tetracarbonsäureestern, Monoestern von gesättigten oder ungesättigten, geradkettigen oder verzweigten aliphatischen C₁₋₅-Monocarbonsäuren und gesättigten oder ungesättigten, geradkettigen oder verzweigten aliphatischen Alkoholen oder Polyolen mit 1 bis 26 Kohlenstoffatomen, Monoestern von gesättigten oder ungesättigten, geradkettigen oder verzweigten aliphatischen C₁₋₂₆-Monocarbonsäuren und gesättigten oder ungesättigten, geradkettigen oder verzweigten aliphatischen C₁₋₅-Alkoholen,
V) nicht filternden Pigmenten, Perlglanzpigmenten und Füllstoffen,
VI) Verbindungen vom Ceramidtyp der allgemeinen Formel (IV): 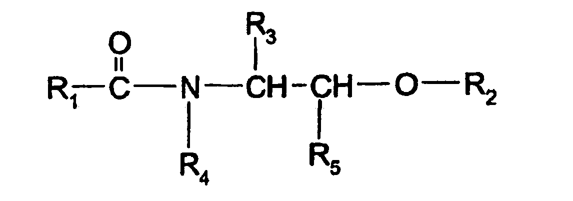
in der bedeuten:
- R₁:
- eine geradkettige oder verzweigte, gesättigte oder ungesättigte C₁₋₅₀-Kohlenwasserstoffgruppe, vorzugsweise eine Gruppe mit 5 bis 50 Kohlenstoffatomen, wobei diese Gruppe mit einer oder mehreren Hydroxygruppe(n), die gegebenenfalls mit einer Säure R₇COOH verestert ist, substituiert sein kann, wobei R₇ eine gesättigte oder ungesättigte, geradkettige oder verzweigte C₁-35-Kohlenwasserstoffgruppe ist, die gegebenenfalls ein-oder mehrfach hydroxyliert ist, wobei die Hydroxygruppe(n) der Gruppe R₇ gegebenenfalls mit einer gesättigten oder ungesättigten, geradkettigen oder verzweigten, gegebenenfalls ein- oder mehrfach hydroxylierten C₁₋₃₅-Fettsäure verestert sein kann;
- eine Gruppe R"-(NR-CO)-R', wobei R Wasserstoff oder eine ein- oder mehrfach hydroxylierte, vorzugsweise einfach hydroxylierte C₁₋₂₀-Kohlenwasserstoffgruppe ist, R' und R" Kohlenwasserstoffgruppen bedeuten, bei denen die Anzahl der Kohlenstoffatome im Bereich von 9 bis 30 liegt, R' eine zweiwertige Gruppe darstellt;
- eine Gruppe R₈-O-CO-(CH₂)ₚ, wobei R₈ eine C₁₋₂₀-Kohlenwasserstoffgruppe bedeutet und p eine ganze Zahl von 1 bis 12 ist;
- R₂ ist unter Wasserstoff, einer Gruppe vom Saccharidtyp, insbesondere einer (Glycosyl)ₙ-, (Galactosyl)ₘ- oder Sulfogalactosylgruppe, einem Sulfat- oder Phosphatrest, einem Phosphorylethylamin- oder Phosphorylethylammoniumrest ausgewählt, wobei n im Bereich von 1 bis 4 und m im Bereich von 1 bis 8 liegt;
- R₃ bedeutet ein Wasserstoffatom oder eine gesättigte oder ungesättigte, hydroxylierte oder nicht hydroxylierte C₁₋₃₃-Kohlenwasserstoffgruppe, wobei die Hydroxygruppe(n) mit einer anorganischen Säure oder einer Säure R₇COOH verestert sein können, wobei R₇ die oben angegebenen Bedeutungen hat, die Hydroxygruppe(n) mit (Glycosyl)ₙ, (Galactosyl)ₘ, Sulfogalactosyl, Phosphorylethylamin oder Phosphorylethylammonium verethert sein können und R₃ auch mit einer oder mehreren C₁₋₁₄-Alkylgruppen substituiert sein kann;
vorzugsweise bedeutet R₃ eine C₁₅₋₂₆-α-Hydroxyalkylgruppe, wobei die Hydroxygruppe gegebenenfalls mit einer C₁₆₋₃₀-α-Hydroxysäure verestert ist;
- R₄ bedeutet ein Wasserstoffatom, Methyl, Ethyl, eine gesättigte oder ungesättigte, geradkettige oder verzweigte C₃₋₅₀- Kohlenwasserstoffgruppe, die gegebenenfalls hydroxyliert ist oder eine Gruppe CH₂-CHOH-CH₂O-R₆, in der R₆ eine C₁₀₋₂₆-Kohlenwasserstoffgruppe oder eine Gruppe R₈-O-CO-(CH₂)ₚ bedeutet, wobei R₈ eine C₁₋₂₀-Kohlenwasserstoffgruppe ist, und p im Bereich von 1 bis 12 liegt,
- R₅ bedeutet Wasserstoff oder eine gesättigte oder ungesättigte, geradkettige oder verzweigte C₁₋₃₀-Kohlenwasserstoffgruppe, die gegebenenfalls ein- oder mehrfach hydroxyliert ist, wobei die Hydroxygruppe(n) mit einer (Glycosyl)n, (Galactosyl)m, Sulfogalactosyl, Phosphorylethylamin oder Phosphorylethylammonium-Gruppe verethert sein können,
mit der Maßgabe, dass R₄ nicht Wasserstoff, Methyl oder Ethyl bedeutet, wenn R₃ und R₅ Wasserstoff oder wenn R₃ Wasserstoff und R₅ Methyl bedeutet.

2. Zusammensetzung nach Anspruch 1, **dadurch gekennzeichnet, dass** der hydrophobe Teil des amphiphilen Polymers 6 bis 50 Kohlenstoffatome umfasst.

3. Zusammensetzung nach Anspruch 2, **dadurch gekennzeichnet, dass** der hydrophobe Teil des amphiphilen Polymers 6 bis 22 Kohlenstoffatome umfasst.

4. Zusammensetzung nach Anspruch 3, **dadurch gekennzeichnet, dass** der hydrophobe Teil des amphiphilen Polymers 6 bis 18 Kohlenstoffatome umfasst.

5. Zusammensetzung nach Anspruch 4, **dadurch gekennzeichnet, dass** der hydrophobe Teil des amphiphilen Polymers 12 bis 18 Kohlenstoffatome umfasst.

6. Zusammensetzung nach einem der Ansprüche 1 bis 5, **dadurch gekennzeichnet, dass** die amphiphilen Polymere ganz oder teilweise mit einer anorganischen oder organischen Base neutralisiert werden.

7. Zusammensetzung nach einem der Ansprüche 1 bis 6, **dadurch gekennzeichnet, dass** die amphiphilen Polymere eine zahlenmittlere Molmasse von 1 000 bis 20 000 000 g/mol haben.

8. Zusammensetzung nach Anspruch 7, **dadurch gekennzeichnet, dass** die zahlenmittlere Molmasse im Bereich von 20 000 bis 5 000 000 g/mol liegt.

9. Zusammensetzung nach Anspruch 8, **dadurch gekennzeichnet, dass** das zahlenmittlere Molekulargewicht in einem Bereich von 100 000 bis 1 500 000 g/mol liegt.

10. Zusammensetzung nach einem der vorhergehenden Ansprüche, **dadurch gekennzeichnet, dass** die wässrige Lösung mit 1 Gew.-% der Polymere bei einer Temperatur von 25 °C eine Viskosität von 20 000 bis 100 000 mPas·s hat, wobei die Viskosität mit einem Brookfield-Viskosimeter, Nadel 7, gemessen wird.

11. Zusammensetzung nach einem der vorhergehenden Ansprüche, **dadurch gekennzeichnet, dass** die amphiphilen Polymere durch radikalische Polymerisation mit Fällung in *t*-Butanol hergestellt sind.

12. Zusammensetzung nach einem der vorhergehenden Ansprüche, **dadurch gekennzeichnet, dass** die amphiphilen Polymere vernetzt oder nicht vernetzt sind.

13. Zusammensetzung nach Anspruch 12, **dadurch gekennzeichnet, dass** die amphiphilen Polymere vernetzt sind.

14. Zusammensetzung nach Anspruch 13, **dadurch gekennzeichnet, dass** das oder die Vernetzungsmittel unter den Verbindungen mit mehreren olefinisch ungesättigten Bindungen ausgewählt sind.

15. Zusammensetzung nach Anspruch 14, **dadurch gekennzeichnet, dass** das oder die Vernetzungsmittel unter Methylen-bisacrylamid, Allylmethacrylat oder Trimethylolpropantriacrylat (TMPTA) ausgewählt sind.

16. Zusammensetzung nach einem der Ansprüche 13 bis 15, **dadurch gekennzeichnet, dass** der Vernetzungsgrad vorzugsweise im Bereich von 0,01 bis 10 Mol-% und insbesondere im Bereich von 0,2 bis 2 Mol-%, bezogen auf das Polymer, liegt.

17. Zusammensetzung nach einem der Ansprüche 1 bis 16, **dadurch gekennzeichnet, dass** die hydrophobe Gruppe R₂ der Formel (I) unter den geradkettigen, verzweigten oder cyclischen C₆₋₁₈-Alkylgruppen; perfluorierten Alkylgruppen mit 6 bis 18 Kohlenstoffatomen; der Cholesterylgruppe oder einem Cholesterinester oder aromatischen, polycyclischen Gruppen ausgewählt ist.

18. Zusammensetzung nach einem der Ansprüche 1 bis 17, **dadurch gekennzeichnet, dass** die polyalkoxylierte Kette aus Ethylenoxideinheiten und/oder Propylenoxideinheiten besteht.

19. Zusammensetzung nach Anspruch 18, **dadurch gekennzeichnet, dass** die polyalkoxylierte Kette ausschließlich aus Ethylenoxideinheiten besteht.

20. Zusammensetzung nach einem der Ansprüche 1 bis 19, **dadurch gekennzeichnet, dass** die Anzahl der alkoxylierten Einheiten im Bereich von 3 bis 50 liegt.

21. Zusammensetzung nach Anspruch 20, **dadurch gekennzeichnet, dass** die Anzahl der alkoxylierten Einheiten im Bereich von 7 bis 25 liegt.

22. Zusammensetzung nach den Ansprüchen 1 bis 21, **dadurch gekennzeichnet, dass** das amphiphile AMPS-Polymer unter den Copolymeren ausgewählt ist, die aus 2-Acrylamido-2-methylpropan-sulfonsäure(AMPS)-Einheiten der folgenden Formel (II): 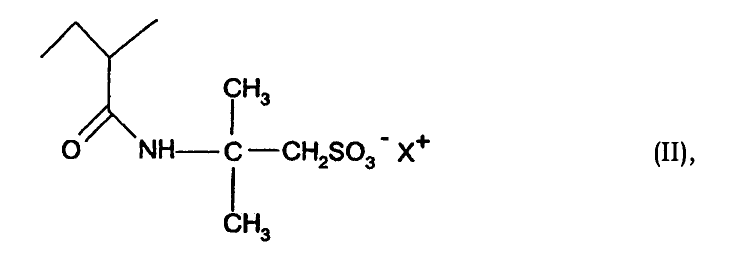 in der X⁺ ein Proton, ein Alkalimetallkation, ein Erdalkalimetallkation oder ein Ammoniumion ist,
und aus den Einheiten der folgenden Formel (III) bestehen: 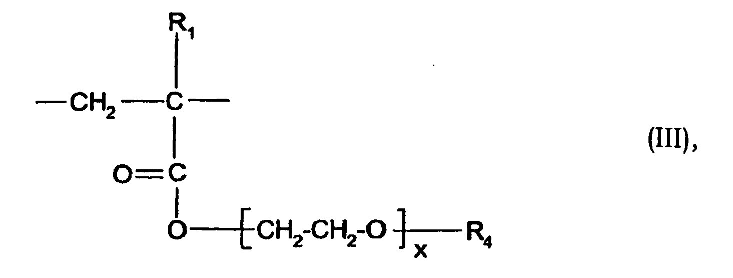 worin bedeuten:
x eine ganze Zahl im Bereich von 3 bis 100, vorzugsweise von 5 bis 80 und noch bevorzugter von 7 bis 25;
R₁ die in der Formel (I) angegebenen Bedeutungen und
R₄ eine geradkettige oder verzweigte C₆₋₂₂-Alkylgruppe und noch bevorzugter eine C₁₀₋₂₂-Alkylgruppe.

23. Zusammensetzung nach Anspruch 22, **dadurch gekennzeichnet, dass** x 25, R₁ Methyl und R₄ n-Dodecyl ist.

24. Zusammensetzung nach den Ansprüchen 1 bis 23, **dadurch gekennzeichnet, dass** der prozentuale molare Anteil der Einheiten der Formel (I) oder der Einheiten der Formel (III) in den Polymeren im Bereich von 50,1 bis 99,9 % ist.

25. Zusammensetzung nach den Ansprüchen 1 bis 23, **dadurch gekennzeichnet, dass** der prozentuale molare Anteil der Einheiten der Formel (I) oder der Einheiten der Formel (III) in den Polymeren im Bereich von 0, 1 bis 50 % liegt.

26. Zusammensetzung nach einem der vorhergehenden Ansprüche, **dadurch gekennzeichnet, dass** die amphiphilen Polymere in Konzentrationen von 0,01 bis 30 Gew.-%, vorzugsweise von 0,1 bis 10 Gew.-%, noch bevorzugter von 0,1 bis 5 Gew.-%, und insbesondere von 0,5 bis 2 Gew.-%, bezogen auf das Gesamtgewicht der Zusammensetzung, enthalten sind.

27. Zusammensetzung nach dem vorhergehenden Anspruch, **dadurch gekennzeichnet, dass** die synthetischen Öle Polyolefine vom Polybutentyp, die gegebenenfalls hydriert sind, oder vom Polydecentyp sind, die gegebenenfalls hydriert sind.

28. Zusammensetzung nach einem der vorhergehenden Ansprüche, **dadurch gekennzeichnet, dass** die natürlichen oder synthetischen etherische Öle unter Eucalyptusöl, Lavandinöl, Lavendelöl, Vetiveröl, Litsea-cubeba-Öl, Citronenöl, Sandelholzöl, Rosmarinöl, Kamillenöl, Bergbohnenkrautöl, Muskatnussöl, Zimtöl, Y-sopöl, Kümmelöl, Orangenöl, Geraniolöl, Wacholderöl oder Bergamottöl ausgewählt sind.

29. Zusammensetzung nach einem der vorhergehenden Ansprüche, **dadurch gekennzeichnet, dass** die pflanzlichen Wachse unter Carnaubawachs, Candelillawachs, Olivenwachs, Reiswachs, gehärtetem Jojobawachs oder den reinen Blütenwachsen, wie e-therisches Wachs der Cassisblume, Wachsen marinen Ursprungs ausgewählt sind.

30. Zusammensetzung nach einem der vorhergehenden Ansprüche, **dadurch gekennzeichnet, dass** die Verbindungen vom Ceramidtyp unter:
- 2-N-Linoleoylamino-octadecan-1,3-diol,
- 2-N-Oleoylamino-octadecan-1,3-diol,
- 2-N-Palmitoylamino-octadecan-1, 3-diol,
- 2-N-Stearoylamino-octadecan-1,3-diol,
- 2-N-Behenoylamino-octadecan-1,3-diol,
- 2-N-[2-Hydroxypalmitoyl]-amino-octadecan-1,3-diol,
- 2-N-Stearoylamino-octadecan-1,3,4-triol und insbesondere N-Stearoylphytosphingosin,
- 2-N-Palmitoylamino-hexadecan-1,3-diol,
- (Bis(N-hydroxyethyl-N-cetyl)malonamid),
- N-(2-hydroxyethyl)-N-(3-cetyloxy-2-hydroxypropyl)-cetylsäureamid,
- N-Docosanoyl-N-methyl-D-glucamin
und den Gemischen dieser Verbindungen ausgewählt sind.

31. Zusammensetzung nach einem der vorhergehenden Ansprüche, **dadurch gekennzeichnet, dass** die Ester unter Ethylpalmitat, Isopropylpalmitat, Isopropylmyristat, Butylmyristat, Butylstearat, Isobutylstearat oder Dioctylmalat ausgewählt sind.

32. Zusammensetzung nach einem der vorhergehenden Ansprüche, **dadurch gekennzeichnet, dass** die Pigmente unter Titandioxid, das gegebenenfalls oberflächenbehandelt ist, Oxiden von Zirconium oder Cer, sowie den Oxiden von Zink, Eisen oder Chrom, Manganviolett, Ultramarin und Eisenblau, Rußschwarz, und den Lacken auf Basis von Cochenillekarmin, Barium, Strontium, Calcium oder Aluminium, weißen Perlglanzpigmenten, wie Titanglimmer-Pigmenten, von Bismutoxidchlorid-Glimmer-Pigmenten, farbigen Perlglanzpigmenten, wie Titanglimmer-Pigmenten mit Eisenoxiden, Titanglimmerpigmenten insbesondere mit Eisenblau oder Chromoxid, Titanglimmerpigmenten mit einem organischen Pigment des vorangegangenen Typs ausgewählt sind.

33. Zusammensetzung nach einem der vorhergehenden Ansprüche, **dadurch gekennzeichnet, dass** die Füllstoffe mineralisch oder organisch, lamellar oder sphärisch sind.

34. Zusammensetzung nach dem vorangegangenen Anspruch, **dadurch gekennzeichnet, dass** die Füllstoffe unter Talk, Glimmer, Siliciumdioxid, Kaolin, Nylonpulver^{®}, Poly-β-Alaninpulver und Polyethylenpulver, Teflon^{®}, Lauroyllysin, Stärke, Bornitrid, Pulvern aus Tetrafluorethylenpolymeren, hohlen Mikrokugeln und Siliconharz-Mikrokugeln, gefälltem Calciumcarbonat, Magnesiumcarbonat und Magnesiumhydrogencarbonat, Siliciumdioxid-Mikrohohlkugeln, Mikrokapseln aus Glas oder Keramik ausgewählt sind.

35. Zusammensetzung nach einem der vorhergehenden Ansprüche, **dadurch gekennzeichnet, dass** die in Wasser unlöslichen Verbindungen in einer Konzentration von 0,001 bis 40 Gew.-%, bezogen auf das Gesamtgewicht der Zusammensetzung, vorliegen.

36. Zusammensetzung nach einem der vorhergehenden Ansprüche, **dadurch gekennzeichnet, dass** das physiologisch akzeptable Milieu aus Wasser oder Wasser und mindestens einem organischen Lösungsmittel besteht, das unter hydrophilen organischen Lösungsmitteln, lipophilen organischen Lösungsmitteln, amphiphilen Lösungsmitteln und ihren Gemischen ausgewählt ist.

37. Zusammensetzung nach einem der vorhergehenden Ansprüche, **dadurch gekennzeichnet, dass** sie als Creme zur Gesichtspflege, Körpermilch, Duschgel, Badegel, als Zusammensetzung zum Färben von Haaren, Zusammensetzung zum dauerhaften Verformen der Haare, Zusammensetzung zum Reinigen und/oder zur Abschminken des Gesichts, als Haarwaschmittel, oder als ein nach dem Haarewaschen anzuwendendes Mittel vorliegt.

38. Kosmetische Verwendung der Zusammensetzung nach einem der Ansprüche 1 bis 37 zur Behandlung und Pflege der Haut des Gesichts und/oder des Körpers, der Schleimhäute, der Kopfhaut und/oder der Haare.

39. Verwendung der Zusammensetzung nach einem der Ansprüche 1 bis 37 zur Herstellung einer Zusammensetzung, die dazu bestimmt ist, empfindliche Haut und/ oder empfindliche Kopfhaut zu behandeln.

40. Verfahren zur kosmetischen Behandlung der Haut, der Kopfhaut, der Haare, der Nägel und/oder der Schleimhäute, **dadurch gekennzeichnet, dass** eine Zusammensetzung nach einem der Ansprüche 1 bis 37 auf die Haut, die Kopfhaut, die Haare, die Nägel und/oder die Schleimhäute aufgetragen wird.

41. Verwendung eines amphiphilen Polymers, das mindestens ein Monomer mit einer ethylenisch ungesättigten Bindung und mit einer in freier oder teilweise oder vollständig neutralisierter Form vorliegenden Sulfonsäuregruppe, das ferner mindestens einen hydrophoben Teil aufweist, nach einem der Ansprüche 1 bis 26, um eine kosmetische oder dermatologische Zusammensetzung, die mindestens eine in Wasser unlösliche Verbindung enthält, zu stabilisieren und/oder ihre Abscheidung zu verbessern.
